# EUROPÄISCHE PATENTSCHRIFT

(11) **EP 0 616 801 B1**
(45) Veröffentlichungstag und Bekanntmachung des Hinweises auf die Patenterteilung: **27.05.1998**
(21) Anmeldenummer: 94810161.3
(22) Anmeldetag: 15.03.1994
(51) Int. Cl.: A61K 9/127

(54) **Verfahren zur Herstellung einer Liposomendispersion im Hochdruckbereich**
Process for preparing a liposome dispersion at high pressure
Procédé pour la préparation à haute pression d'une dispersion de liposomes

(30) Priorität: 24.03.1993 CH 891/93
(43) Veröffentlichungstag der Anmeldung: 28.09.1994
(73) Patentinhaber: Novartis AG, 4058 Basel (CH)
(72) Erfinder: Frederiksen, Lene, CH-4057 Basel (CH); Anton, Klaus, Dr., CH-4057 Basel (CH); van Hoogevest, Peter, Dr., CH-4125 Riehen (CH)

(56) Entgegenhaltungen:
- WO-A-88/01864
- FR-A- 2 550 706

## Beschreibung

Die vorliegende Erfindung betrifft ein neues, vorteilhaftes Verfahren zur Herstellung einer Liposomendispersion.

Liposomendispersionen mit verschiedenen Einschlussverbindungen und Phospholipiden, wie Lecithin, sind in zahlreichen Publikationen beschrieben und bereits klinisch erprobt worden. Zur Illustration des Standes der Technik wird die Europäische Patentanmeldung (im folgenden mit EP-A bezeichnet) 178 624 genannt, worin eine Liposomendispersion mit synthetischem, gereinigtem Natrium-1,2-di-(9-cis-octadecenoyl)-3-sn-phosphatidyl-S-serin und 1-n-Hexadecanoyl-2-(9-cis-octadecenoyl)-3-sn-phosphatidylcholin als Phospholipide und lipophilem N-Acetyl-D-muramyl-L-alanyl-D-isoglutaminyl-L-alanin-2-(1,2-dipalmitoyl-sn-glycero-3-hydroxyphosphoryloxy)-ethylamid oder hydrophilem Doxorubicin als verkapselte Wirkstoffe beschrieben ist. Diese Dispersionen sind u.a. intravenös applizierbar.

Bekannt sind auch Liposomendispersionen mit Einschlussverbindungen ohne eigentliche pharmakologische Eigenschaften wie Zink-Phthalocyanin, radioaktive Markerverbindungen oder fluoreszierende Verbindungen. Zur Illustration ist die EP-A-451 103 genannt, worin eine Liposomendispersion mit Zinkphthalocyanin beschrieben ist, die nach intravenöser Applikation erst unter Anregung mit gebündeltem Licht (LASER) in der sogenannten Photodynamischen Chemotherapie anwendbar ist.

In der Literatur sind zahlreiche Verfahren zur Herstellung von Liposomendispersionen beschrieben, z.B. durch Behandlung einer wässrigen Phospholipiddispersion mit Ultraschall; Dispersion von Phospholipiden mit Tensiden in wässriger Phase und Entfernung der Tenside durch Dialyse; Auflösen von Phospholipiden in organischen Lösungsmitteln, Entfernen des Lösungsmittels durch Lyophilisierung und Dispersion des Rückstandes in wässriger Phase; Infusionsmethoden oder Umkehrphasenverdampfung.

Viele bekannte Herstellungsverfahren sind nachteilig, da nur ein Bruchteil der verwendeten Phospholipidmenge Liposomen bildet und diese Liposomen ebenfalls nur einen Bruchteil an Einschlussverbindung enthalten. Es können sich zusätzlich auch noch Mischmizellen, Gelstrukturen und Doppelschichtaggregate undefinierbarer Grösse bilden. Bekannt sind ferner Stabilitätsprobleme, stark variierende Grössenverteilung der Liposomen, mangelnde Reproduzierbarkeit der Verfahren selbst, hohe Restmengen an organischen Lösungsmitteln, Restgehalt an Tensiden etc..

Sämtlichen bisher bekannten Herstellungsverfahren ist ausserdem gemeinsam, dass nur ein geringer Anteil des zu verkapselnden Stoffes oder Stoffgemisches in der Doppelschichtmembran oder im Innenraum der Liposomen tatsächlich verkapselt wird. Durch Wahl eines lipophilen Stoffs oder Stoffgemischs lässt sich die verkapselte Wirkstoffmenge steigern. Will man dagegen wasserlösliche oder hydrophile Stoffe verkapseln, bleibt der Anteil an verkapselten Stoffen im Vergleich zur eingesetzten Gesamtmenge stets gering. Wasserlösliche oder hydrophile Stoffe neigen kaum dazu, sich aus der wässrigen Phase in einer Lipidphase anzureichern. Ausserdem ist die Stabilität von Lipidmembranen gering. Bei deren Öffnung wird der wässrige Inhalt des Innenraumes der Liposomen mit der die Liposomen umgebenden wässrigen Phase ausgetauscht, so dass der Anreicherungsgrad eines wasserlöslichen Wirkstoffs in den Liposomen sinkt.

Der vorliegenden Erfindung liegt die Aufgabe zugrunde, ein neues, verbessertes Verfahren zur Herstellung von Liposomen zum Einschluss von wasserlöslichen oder hydrophilen Stoffen oder Stoffgemischen bereitzustellen, welches im Vergleich mit bekannten Verfahren den überraschenden Vorteil der Steigerung des Anteils an tatsächlich eingeschlossenen Stoffen oder Stoffgemischen und bei pharmazeutischer Anwendung den Vorteil möglichst steriler Arbeitsbedingungen bietet.

Diese Aufgabe wird durch die vorliegende Erfindung gelöst, welche ein vorteilhaftes Verfahren zur Herstellung einer Liposomendispersion betrifft. Die Liposomendispersion enthält
a) einen in Liposomen zu verkapselnden Stoff oder ein zu verkapselndes Stoffgemisch mit wasserlöslichen oder hydrophilen Eigenschaften;
b) mindestens ein Phospholipid der Formel 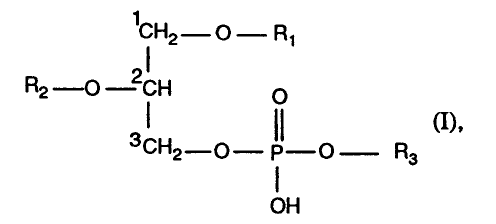 worin R₁ C₁₀₋₂₀-Acyl, R₂ Wasserstoff oder C₁₀₋₂₀-Acyl, R₃ Wasserstoff, 2-Trimethylamino-1-ethyl, 2-Amino-1-ethyl, C₁₋₄-Alkyl, C₁₋₅-Alkyl substituiert durch Carboxy, C₂₋₅-Alkyl substituiert durch Hydroxy, C₂₋₅-Alkyl substituiert durch Carboxy und Hydroxy oder C₂₋₅-Alkyl substituiert durch Carboxy und Amino bedeuten oder Salze dieser Verbindungen;
c) Wasser in der für die vorgesehene Anwendung erforderlichen Reinheit und gegebenenfalls
d) zusätzliche für die vorgesehene Anwendung übliche Hilfsstoffe.

Das Verfahren gemäss vorliegender Erfindung ist dadurch gekennzeichnet, dass man ein Gemisch bestehend aus mindestens einem Phospholipid b) und gegebenenfalls für die vorgesehene Anwendung üblichen lipophilen Hilfsstoffen d) einer mobilen Trägerphase bestehend aus Kohlendioxid und einem polaren organischen Lösungsmittel (Modifikator) unter Druck- und Temperaturbedingungen höher als dem kritischen Druck und der kritischen Temperatur einer reinen Kohlendioxidphase aussetzt, die erhältliche komprimierte Mischphase auf Normaldruck entspannt und in eine wässrige Phase enthaltend einen in Liposomen zu verkapselnden Stoff oder ein zu verkapselndes Stoffgemisch mit wasserlöslichen oder hydrophilen Eigenschaften a) und gegebenenfalls für die vorgesehene Anwendung übliche wasserlösliche Hilfsstoffe d) überführt und gegebenenfalls das organische Lösungsmittel entfernt und/oder eine Fraktion von Liposomen mit einem gewünschten Bereich des Durchmessers abtrennt und/oder die Liposomendispersion in eine für die vorgesehene Anwendung geeignete Form bringt.

In einer besonders bevorzugten Verfahrensvariante wird das Phospholipid 1-n-Hexadecanoyl-2-(9-cis-octadecenoyl)-3-sn-phosphatidylcholin (POPC) mit dem lipophilen Hilfsstoff Cholesterin einer mobilen Trägerphase bestehend aus Kohlendioxid mit ca. 5-7 % Ethanol als Modifikator oberhalb des für eine reine CO₂-Phase geltenden kritischen Drucks und kritischer Temperatur (≥ 72 bar, ≥ 32°C) ausgesetzt. Diese komprimierte Mischphase wird nach ihrem Entspannen auf Normaldruck mit einer wässrigen Vorlage versetzt, welche einen wasserlöslichen Wirkstoff wie EDATREXATE (10-EDAM) enthält. Dabei bilden sich spontan Liposomen mit einem hohen Anteil dieser wasserlöslichen Einschlussverbindung.

Die weiter vorn und im folgenden genannten Begriffe werden im Rahmen der Beschreibung der Erfindung wie folgt definiert:

Der Begriff: Ein in Liposomen zu verkapselnder Stoff oder ein zu verkapselndes Stoffgemisch mit wasserlöslichen oder hydrophilen Eigenschaften - definiert hydrophile oder wasserlösliche wasserlösliche Stoffe und Stoffgemische des Standes der Technik, deren Fähigkeit bekannt ist, in Liposomen mit Phospholipiddoppelschichten eingeschlossen zu werden.

Liposomen sind in der Literatur in zahlreichen Veröffentlichungen beschrieben worden. Ihr Aufbau und ihre Verwendung sind Gegenstand vieler Untersuchungen. Man unterscheidet unilamellare Liposomen mit einer Doppelschicht von multilamellaren Liposomen mit mehreren Doppelschichten aus Phospholipiden, die zwiebelschalenförmig angeordnet sind. Die Grössenordnung der Liposomen ist variabel zwischen ca. 1,0 x 10⁻⁸ bis ca. 1,0 x 10⁻⁵ m.

Die therapeutische Verwendung von Liposomen als Träger insbesondere von lipophilen pharmazeutischen Wirkstoffen ist bekannt. Ferner sind Liposomen als Träger von anderen lipophilen Stoffen mit biologischer Wirkung wie Proteinen, z.B. Antikörpern oder Enzymen, Hormonen, Vitaminen oder Genen, oder zu analytischen Zwecken als Träger von markierten Verbindungen vorgeschlagen worden.

Liposomen und ihre Herstellung sind in dem Uebersichtswerk von Gregoriadis G. (Herausgeber) Liposome Technology, Vol. II, Incorporation of Drugs, Proteins and Genetic Material, CRC Press 1984, beschrieben.

Bei dem in Liposomen zu verkapselnden Stoff oder Stoffgemisch unterscheidet man zwischen seinen hydrophilen oder wasserlöslichen Eigenschaften. Als hydrophile Eigenschaft eines Stoffs oder Stoffgemisches versteht man die auch bei Tensiden bekannte Neigung, sich in der Phasengrenzfläche des Wassers anzureichern. Dies setzt die Gegenwart von sogenannten hydrophilen Gruppen in der Molekularstruktur des betreffenden Stoffs oder Stoffgemisches voraus, welche mit Wasser Wechselwirkungen im Sinne einer Anziehung eingehen können.

Ein Stoff oder Stoffgemisch wird als wasserlöslich definiert, wenn ein partieller oder vollständiger Lösungsvorgang seiner Einwaage in der wässrigen Phase stattgefunden hat und eine Teilmenge oder die Gesamtmenge des betreffenden Stoffs oder das Stoffgemisches im Lösungsmittel Wasser in molekulardisperser Verteilung vorliegt. Für pharmazeutische Zwecke genügt die für die Wirksamkeit erforderliche Mindestkonzentration an gelöstem Wirkstoff in Wasser, gegebenenfalls in kolloiddisperser Verteilung, wobei keine Sedimentation von Stoffen stattfinden soll.

Schwerlösliche Wirkstoffe kann man in wasserlösliche, pharmazeutisch annehmbare Salze überführen, z.B. in ein Hydrobromid, Hydrochlorid, Mesylat, Acetat, Succinat, Lactat, Tartrat, Fumarat, Sulfat, Maleat, etc. und für das Verfahren anwendbar machen. Ferner lassen sich schwerlösliche Wirkstoffe durch Überführung in wasserlösliche Derivate bzw. Zusatz der im folgenden genannten Löslichkeitsvermittler (Solubilisatoren) wasserlöslich machen.

Geeignete pharmazeutische Wirkstoffe sind in wasserlöslicher Form, z.B. als wasserlösliche Salze, vorliegende oder durch Zusatz von Solubilisatoren wasserlöslich gemachte entzündungshemmende Mittel, z.B. Dexamethason, Natriumdexamethasonsulfat, Hydrocortison oder Prednisolen, Coronardilatatoren, z.B. Nifedipin, Isosorbitdinitrat, Nitroglycerin, Dilthiazem, Trapidil, Dipyridamol oder Dilazep, Prostaglandine, z.B. Prostaglandin E₁, E₂ oder F_{2α}, periphere Vasodilatatoren, z.B. Ifenprodil, Cinepazetmaleat, Cyclandelat, Cinnarizin oder Pentoxyphyllin, Antibiotica, z.B. Ampicillin, Amoxycillin, Cephalexin, Cefradin, Cefroxadin, Cefaclor, Erythromycin, Bacampicillin, Minocyclin oder Chloramphenicol, krampflösende Mittel, z.B. Propanthelin, Atropin oder Scopolamin, Antitussiva und Antiasthmatica, z.B. Theophyllin, Aminophyllin, Methylephedrin, Procatechol, Trimethoquinol, Codein, Clofedanolol oder Dextromethorphan, Diuretica, z.B. Furosemid oder Acetazolamid, muskelentspannende Mittel, z.B. Chlorphenesincarbamat, Tolperison, Eperison oder Baclofen, schwache Beruhigungsmittel, z.B. Oxazolam, Diazepam, Clotiazepam, Medazepam, Temazepam oder Fludiazepam, starke Beruhigungsmittel, z.B. Sulpirid, Clocapramin oder Zotepin, Beta-Blocker, z.B. Pindolol, Propranolol, Carteolol, Oxprenolol, Metoprolol oder Labetalol, Antiarrhythmica, z.B. Procainamid, Disopyramid, Ajimalin oder Quinidin, Antigichtmittel wie Allopurinol, Anticoagulanzien wie Ticlopidin, Antiepileptica, z.B. Phenytoin oder Valproat, Antihistaminica, z.B. Chlorpheniramin, Clemastin, Mequitazin, Alimemazin, Cyproheptadin, Mittel gegen Uebelkeit und Schwindel, z.B. Diphenidol, Methochlopromid, Domperidon oder Bethahistin, Blutdrucksenkende Mittel, z.B. Reserpin, Rescinnamin, Methyldopa, Prazosin, Clonidin oder Budralazin, Sympathomimetica, z.B. Dihydroergotamin, Isoproterenol oder Etilefrin, Expectoranzien, z.B. Bromhexin, Carbocistein, L-Ethylcystein oder L-Methylcystein, orale Antidiabetica, z.B. Glibencamid oder Tolbutamid, kardiovaskuläre Mittel, z.B. Ubidecarenon oder Adenosin.

Bevorzugt sind Immunsuppressive, wie Ciclosporin, Zytostatika, wie EDATREXATE (10-EDAM), Doxorubicin, Cytarabin, Trifosamid, Cyclophosphamid, Fluorouracil oder Methotrexat, sowie wasserlösliche Sulfoderivate von Phthalocyanin, z.B. Tetrasulfophtalocyanin, welche in der photodynamischen Chemotherapie verwendbar sind.

Die Liposomendispersion kann anstelle eines pharmazeutischen Wirkstoffs oder einer Wirkstoffkombination auch andere zu verkapselnde Stoffe wie radioaktive Markerverbindungen oder fluoreszierende Verbindungen enthalten.

Die Nomenklatur der Phospholipide (I) und die Bezifferung der C-Atome erfolgt anhand der in Eur. J. of Biochem. 79, 11-21 (1977) "Nomenclature of Lipids" von der IUPAC-IUB Commission on Biochemical Nomenclature (CBN) gegebenen Empfehlungen (sn-Nomenklatur, stereospecific numbering).

R₁ und R₂ mit den Bedeutungen C₁₀₋₂₀-Acyl sind vorzugsweise geradkettiges C₁₀₋₂₀-Alkanoyl mit einer geraden Anzahl an C-Atomen und geradkettiges C₁₀₋₂₀-Alkenoyl mit einer Doppelbindung und einer geraden Anzahl an C-Atomen.

Geradkettiges C₁₀₋₂₀-Alkanoyl R₁ und R₂ mit einer geraden Anzahl an C-Atomen sind beispielsweise n-Dodecanoyl, n-Tetradecanoyl, n-Hexadecanoyl oder n-Octadecanoyl.

Geradkettiges C₁₀₋₂₀-Alkenoyl R₁ und R₂ mit einer Doppelbindung und einer geraden Anzahl an C-Atomen sind beispielsweise 6-cis-, 6-trans-, 9-cis- oder 9-trans-dodecenoyl, -tetradecenoyl, -hexadecenoyl, -octadecenoyl oder -icosenoyl, insbesondere 9-cis-octadecenoyl (Oleoyl).

Ein Phospholipid (I), worin R₃ 2-Trimethylamino- 1-ethyl bedeutet, wird mit dem Trivialnamen Lecithin und ein Phospholipid (I), worin R₃ 2-Amino-1-ethyl bedeutet, mit dem Trivialnamen Kephalin bezeichnet. Geeignet sind beispielsweise natürlich vorkommendes Kephalin oder Lecithin, z.B. Kephalin oder Lecithin aus Sojabohnen oder Hühnerei, mit verschiedenen oder identischen Acylgruppen R₁ und R₂ oder Mischungen davon.

Bevorzugt sind synthetische, im wesentlichen reine Phospholipide(I) mit verschiedenen oder identischen Acylgruppen R₁ und R₂.

Der Begriff "synthetisches" Phospholipid (I) definiert Phospholipide, welche bezüglich R₁ und R₂ eine einheitliche Zusammensetzung haben. Solche synthetischen Phospholipide sind vorzugsweise die weiter vorn definierten Lecithine und Kephaline, deren Acylgruppen R₁ und R₂ eine definierte Struktur haben und von einer definierten Fettsäure mit einem Reinheitsgrad höher als ca. 95 % abgeleitet sind. R₁ und R₂ können gleich oder verschieden und ungesättigt oder gesättigt sein. Bevorzugt ist R₁ gesättigt, z.B. n-Hexadecanoyl, und R₂ ungesättigt, z.B. 9-cis-Octadecenoyl (= Oleoyl).

Der Begriff "natürlich vorkommende" Phospholipide (I) definiert Phospholipide, welche bezüglich R₁ und R₂ keine einheitliche Zusammensetzung haben. Solche natürlichen Phospholipide sind ebenfalls Lecithine und Kephaline, deren Acylgruppen R₁ und R₂ strukturell undefinierbar und von natürlich vorkommenden Fettsäuregemischen abgeleitet sind.

Die Forderung "im wesentlichen reines" Phospholipid definiert einen Reinheitsgrad von mehr als 95 % (Gew.) des Phospholipids (I), welcher anhand geeigneter Bestimmungsmethoden, z.B. papierchromatographisch, nachweisbar ist.

Besonders bevorzugt sind synthetische, im wesentlichen reine Phospholipide (I), worin R₁ die Bedeutung geradkettiges C₁₀₋₂₀-Alkanoyl mit einer geraden Anzahl an C-Atomen und R₂ die Bedeutung geradkettiges C₁₀₋₂₀-Alkenoyl mit einer Doppelbindung und einer geraden Anzahl an C-Atomen haben.

In einem besonders bevorzugten Phospholipid (I) bedeuten R₁ n-Dodecanoyl, n-Tetradecanoyl, n-Hexadecanoyl oder n-Octadecanoyl und R₂ 9-cis-Dodecenoyl, 9-cis-Tetradecenoyl, 9-cis-Hexadecenoyl, 9-cis-Octadecenoyl oder 9-cis-Icosenoyl.

In einem Phospholipid (I) ist R₃ mit der Bedeutung C₁₋₄-Alkyl beispielsweise Methyl oder Ethyl.

R₃mit den Bedeutungen C₁₋₅-Alkyl substituiert durch Carboxy, C₂₋₅-Alkyl substituiert durch Hydroxy oder C₂₋₅-Alkyl substituiert durch Carboxy oder Hydroxy sind beispielsweise 2-Hydroxyethyl, 2,3-Dihydroxy-n-propyl, Carboxymethyl, 1- oder 2-Carboxyethyl, Dicarboxymethyl, 2-Carboxy-2-hydroxyethyl oder 3-Carboxy-2,3-dihydroxy-n-propyl.

R₃ mit der Bedeutung C₂₋₅-Alkyl substituiert durch Carboxy und Amino ist z.B. 3-Amino-3-carboxy-n-propyl oder 2-Amino-2-carboxy-n-propyl, vorzugsweise 2-Amino-2-carboxyethyl. Phospholipide (I) mit diesen Gruppen können in Salzform, z.B. als Natrium- oder Kaliumsalz, vorliegen.

Ein ganz besonders bevorzugtes Phospholipid (I) ist synthetisches 1-n-Hexadecanoyl-2-(9-cis-octadecenoyl)-3-sn-phosphatidylcholin (POPC) mit einer Reinheit von mehr als 95 %.

Für die Acylreste in den Phospholipiden (I) sind auch die in Klammern angegebenen Bezeichnungen gebräuchlich:

9-cis-Dodecenoyl (Lauroleoyl), 9-cis-Tetradecenoyl (Myristoleoyl), 9-cis-Hexadecenoyl (Palmitoleoyl), 6-cis-Octadecenoyl (Petroseloyl), 6-trans-Octadecenoyl (Petroselaidoyl), 9-cis-Octadecenoyl (Oleoyl), 9-trans-Octadecenoyl (Elaidoyl), 11-cis-Octadecenoyl (Vaccenoyl), 9-cis-Icosenoyl (Gadoleoyl), n-Dodecanoyl (Lauroyl), n-Tetradecanoyl (Myristoyl), n-Hexadecanoyl (Palmitoyl), n-Octadecanoyl (Stearoyl), n-Icosanoyl (Arachidoyl).

Ein Salz des Phospholipids (I) ist vorzugsweise pharmazeutisch annehmbar. Salze definieren sich durch die Existenz von salzbildenden Gruppen im Substituenten R₃ sowie durch die freie Hydroxygruppe am Phosphor. Möglich ist ebenfalls die Bildung von inneren Salzen. Bevorzugt sind Alkalimetallsalze, insbesondere Natriumsalze.

Die Komponente c) - Wasser in der für die vorgesehene Anwendung erforderlichen Reinheit - ist in dem für die jeweilige Verwendung vorgesehenen Reinheitsgrad in der Liposomendispersion enthalten, welches z.B. nach den Vorschriften der nationalen Pharmakopöen keim- und pyrogenfrei gemacht wurde. Beispielsweise findet Wasser für Injektionszwecke oder sterilisiertes Wasser für Injektionszwecke Verwendung.

In der Liposomendispersion können ausserdem weitere Hilfsstoffe d) enthalten sein, welche z.B. zur Herstellung von isotonischen Bedingungen erforderlich sind, z.B. ionische Zusätze wie Kochsalz oder nichtionische Zusätze (Gerüstbildner) wie Sorbit, Mannit oder Glucose oder wasserlösliche Stabilisatoren für die Liposomendispersion wie Lactose, Fructose oder Sucrose. Insbesondere sind diese Zusätze, z.B. Kochsalz oder Mannit, in den vorgeschriebenen Mengen enthalten, welche zur Herstellung von isotonischen Bedingungen der Injektionslösungen erforderlich sind. In einer besonders bevorzugten Ausführungsform des Verfahrens wird die Liposomendispersion mit dem lipophilen Hilfsstoff Cholesterin hergestellt. Dieser Hilfsstoff wird mit den genannten Phospholipiden der mobilen Trägerphase bestehend aus CO₂ und dem Modifikator Ethanol zugesetzt. Bei der Entspannung dieser Mischphase auf Normaldruck in wässriger Phase bilden sich Liposomen, worin der Hilfsstoff Cholesterin in den Doppelschichten bestehend aus Phospholipiden eingebaut ist. Liposomen mit Cholesterin in der Doppelschicht zeichnen sich durch erhöhte Stabilität aus.

Zusätzlich zu den wasserlöslichen Hilfsstoffen können in der Liposomendispersion für flüssige pharmazeutische Formulierungen verwendbare Hilfsstoffe vorhanden sein, welche die Wasserlöslichkeit der genannten Wirkstoffe erhöhen, z.B. Emulgatoren, Netzmittel oder Tenside, insbesondere Emulgatoren wie Ölsäure, nichtionische Tenside vom Typ Fettsäure-Polyhydroxyalkoholester wie Sorbitanmonolaurat, -oleat, -stearat oder - palmitat, Sorbitantristearat oder -trioleat, Polyoxyethylen-Addukte von Fettsäure-Polyhydroxyalkoholestern wie Polyoxyethylen-sorbitanmonolaurat, -oleat, -stearat, -palmitat, -tristearat oder -trioleat, Polyethylenglycol-Fettsäureester wie Polyoxyethylstearat, Polyethylenglycol-400-stearat, Polyethylenglycol-2000-stearat, insbesondere Ethylenoxid-Propylenoxid Blockpolymere vom Typ Pluronic® (Wyandotte Chem. Corp.) oder Synperonic® (ICI).

Der Vorteil des Verfahrens besteht in seiner Eignung, dass man eine grosse Menge eines wasserlöslichen Stoffes, insbesondere eines wasserlöslichen pharmazeutischen Wirkstoffes wie EDATREXATE (10-EDAM), Doxorubicin, Cytarabin oder Trifosamid, in Liposomen verkapseln kann. Die bisher bekannten Herstellungsverfahren sind für wasserlösliche Einschlussverbindungen nachteilig, indem dabei nur geringe Mengen in Liposomen verkapselt werden, während die grössere Menge sich weiterhin in der wässrigen Phase gelöst befindet.

Als Phospholipid verwendet man vorzugsweise die weiter vorn definierten natürlichen oder synthetischen, im wesentlichen reinen Derivate des Lecithins, insbesondere 1-n-Hexadecanoyl-2-(9-cis-octadecenoyl)-3-sn-phosphatidylcholin (POPC). Als üblicher lipophiler Hilfsstoff wird vorzugsweise Cholesterin verwendet. Durch Einbau dieses Hilfsstoffs, welcher ebenfalls in stabilen, natürlichen Membranen vorhanden ist, erhält man Liposomen mit besonders stabiler Struktur, welche bis zu mehreren Monaten lagerstabil bleiben.

Der Begriff mobile Trägerphase bestehend aus Kohlendioxid und einem polaren organischen Lösungsmittel (Modifikator) definiert eine Mischphase, welche aus Kohlendioxid und polarem Lösungsmittel unter Druck- und Temperaturbedingungen in der Nähe oder oberhalb des kritischen Punktes dieser Mischphase besteht. Die anzuwendenden Druck- und Temperaturbedingungen sind höher als dem kritischen Druck und der kritischen Temperatur einer reinen Kohlendioxidphase entspricht (72 bar und 32°C). Als polare organisches Lösungsmittel (Modifikator-Modifier) verwendet man vorzugsweise Ethanol, aber auch Methanol, tert.-Butanol, Isopropanol, n-Propanol, Methylisobutylketon, Aceton etc..

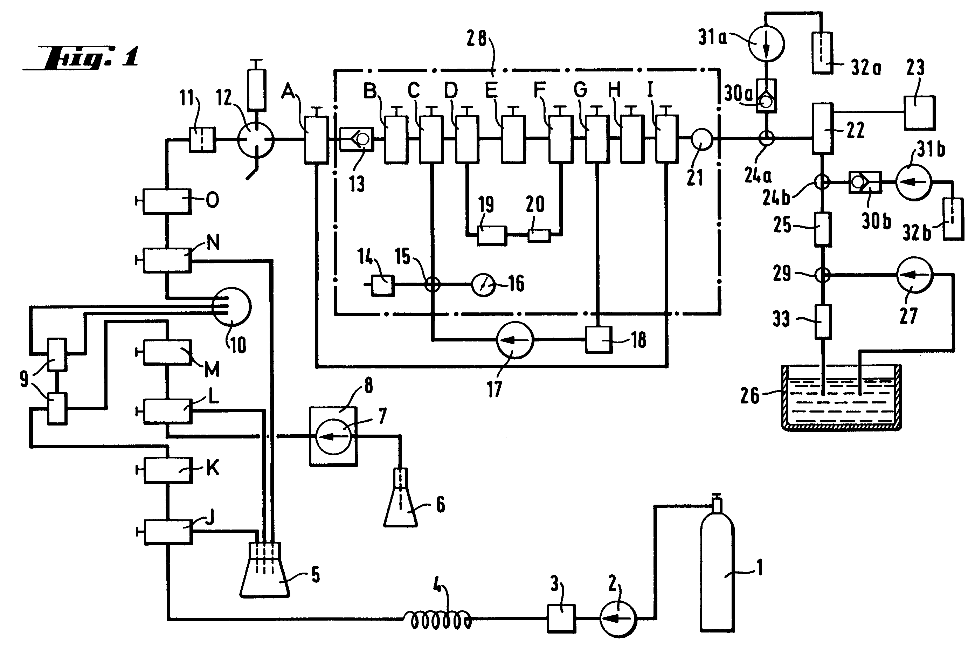

Der Druck in der mobilen Trägerphase bestehend aus Kohlendioxid und dem Modifikator, vorzugsweise Ethanol, wird in einer geschlossenen Apparatur, siehe Fig. 1, auf einen Wert von mindestens 72 bar erhöht. Bevorzugt sind Werte im überkritischen Bereich einer reinen CO₂-Phase bis ca. 1000 bar, vorzugsweise bis ca. 400 bar, insbesondere 200-300 bar. Als Arbeitstemperaturen sind Temperaturen oberhalb Raumtemperatur bis ca. 100°C bevorzugt, insbesondere bei 50-60°C. Die überkritischen Mindestbedingungen für eine reine CO₂-Phase liegen bei ca. 72 bar und 32°C (leicht variierende Angaben in physikochemischen Tabellenwerken aufgrund unterschiedlicher Messmethoden).

Der Begriff komprimierte Mischphase definiert ein homogenes Gemisch unter überkritischen oder angenähert kritischen Druck- und Temperaturbedingungen bestehend aus der mobilen Trägerphase, dem Phospholipid b) und gegebenenfalls für die vorgesehene Anwendung üblichen, lipophilen Hilfsstoffen d), insbesondere Cholesterin.

Die Grösse der gebildeten Liposomen in der wässrigen Phase ist von verschiedenen Rahmenbedingungen abhängig, z.B. der Zusammensetzung der moblien Trägerphase, der Menge des Wirkstoffs und der Lipidkomponenten, deren Mischungsverhältnis und Konzentration in der wässrigen Dispersion, Wahl der Druck- und Temperaturbedingungen, der Durchflussgeschwindigkeit oder Variation der Mischertypen oder Kapillargeometrie, z.B. Länge und Durchmesser der Entspannungskapillare in der für das Verfahren verwendeten Apparatur.

Die für pharmazeutische Anwendungen gegebenenfalls notwendige Entfernung von organischem Lösungsmittel (Modifikator-Modifier) kann nach verschiedenen Verfahren erfolgen, z.B. Abdampfen, Dialyse oder Gelfiltration/Gelchromatographie. Ethanol lässt sich durch Gelfiltration (Sephadex® G 50) aus der wässrigen Lösung entfernen. Bevorzugt entfernt man Ethanol mit CO₂ im Gegenstromprinzip unter Anwendung von überkritischen Druck- und Temperaturbedingungen nach dem in der U.S. Patentschrift 4 492 808 beschriebenen Verfahren.

Man puffert vorzugsweise sauer reagierende wässrige Dispersionen auf pH 7,0 bis 7,8, vorzugsweise 7,2 bis 7,4, ab. Dafür lassen sich vorzugsweise pharmazeutisch annehmbare Pufferlösungen verwenden, deren Herstellung in diversen nationalen Pharmakopöen, z.B. der Europäischen, U.S., Deutschen oder Britischen Pharmakopöe, beschrieben ist. Man kann die Dispersion auch durch Zugabe einer pharmazeutisch annehmbaren, verdünnten wässrigen Base neutralisieren, z.B. verdünnter wässriger Natronlauge. Man neutralisiert üblicherweise unter gleichzeitiger pH-Kontrolle. Gegebenenfalls füllt man mit sterilem, keimfreiem und pyrogenfreiem Wasser auf.

Durch Nachbehandlung der Liposomendispersion, z.B. durch Beschallung mit Ultraschall oder Extrusion durch geradporige Filter (z.B. Nucleopore®), kann man eine besonders einheitliche Grössenverteilung der Liposomen erhalten.

Die Trennung und Isolierung einer Fraktion von grossen Liposomen von einer Fraktion mit kleinen Liposomen, sofern überhaupt notwendig, erfolgt ebenfalls mittels herkömmlicher Trennmethoden, z.B. Gelfiltration oder Ultrafiltration, z.B. mit Sepharose® 4B oder Sephacryl® (Pharmacia SE) als Träger, oder durch Sedimentation der Liposomen in der Ultrazentrifuge, z.B. mit einem Schwerefeld bei 160,000 x g. Beispielsweise setzen sich nach mehrstündigem, z.B. ca. dreistündigem, Zentrifugieren in diesem Schwerefeld Liposomen ab, während die kleinen Liposomen dispergiert bleiben und dekantiert werden können. Nach mehrmaligem Zentrifugieren erreicht man eine vollständige Trennung der grossen von den kleinen Liposomen.

Insbesondere durch Gelfiltration kann man alle in der wässrigen Phase befindlichen Liposomen mit einem Durchmesser grösser als ca. 6,0 x 10⁻⁸ m sowie nicht verkapselte Komponenten und überschüssige, dispergierte Lipide, welche in hochmolekularen Aggregaten vorliegen, abtrennen und so eine wässrige Dispersion mit einer Fraktion von Liposomen mit relativ einheitlicher Grösse herstellen.

Die erfolgte Bildung von Liposomen und ihre Grössenverteilung in wässriger Phase lässt sich in an sich bekannter Weise anhand verschiedener physikalischer Messmethoden nachweisen, z.B. mit gefriergebrochenen (freeze fracture) Proben und Dünnschnitten im Elektronenmikroskop oder durch Röntgendiffraktion, durch dynamische Lichtstreuung, durch Massenbestimmung des Filtrates in der analytischen Ultrazentrifuge und vor allem spektroskopisch, z.B. im Kernresonanzspektrum (¹H, ¹³C und ³¹P).

Die Liposomendispersion ist nach Entfernen von organischen Lösungsmitteln direkt applizierbar, kann aber auch durch Gefrietrocknen in ein Lyophilisat überführt werden, das man unmittelbar vor der Applikation durch Zugabe von Wasser mit dem vorgesehenen Injektionsvolumen rekonstituiert.

Das Verfahren selbst wird in einer geschlossenen Apparatur durchgeführt, welche in der Fig.1 näher illustriert wird. Die Bestandteile der Apparatur sind folgende, die Numerierung der Positionen entspricht den Bezugszeichen in der Zeichnung:

| | | |
|---|---|---|
| 1 | CO₂-Vorratsgefäss | Reines CO₂ (99.9 %) aus Tank mit Tauchrohr (Carbagas CH-Basel). |
| 2 | CO₂-Pumpe | Gilson 306 (Synmedic AG, CH-Zürich) mit Kühlaggregat MGW Lauda RM6, IG Instrumentengesellschaft AG CH-Zürich. |
| 3 | Pumpendrucksicherung | Gilson 805S (Synmedic). |
| 4 | Pulsdämpfer | Reduziert Stösse der Pumpe. Ca. 2 m 1/8-Kapillare im Durchmesser (Supelco SA, CH-Gland). |
| 5 | Abfallgefäss (Waste) | Für Lösungsmittelrückstände. |
| 6 | Modifikator-Vorratsgefäss | (Modifikator) mit Ethanol. |
| 7 | Modifikator-Pumpe mit | Gilson 305 (Synmedic). |
| 8 | Drucksicherung für Pumpe | in Gilson 305 eingebaut. |
| 9 | Statischer Mischer | Lee Visco Mixer, Lee TCMA 2520113T 648430, 2 Stück, Lee Hydraulische Miniaturkomponenten D-Frankfurt. |
| 10 | Dynamischer Mischer | Gilson 811B (Synmedic). |
| 11 | Filter | SSI 05-0150 (Supelco). |
| 12 | Injektor | Gilson 231 und Gilson Dilutor 401 (Synmedic). |
| 13 | Rückschlagsventil | Spectra-Physics Checkvalve mit Hülse (Ciba-Geigy,CH-Basel). (Spectra Physics, CH-Allschwil). |
| 14 | Drucksicherheitsventil | No.20631 25, 2500-25000 Psig (Haskel Inc. Burbank Ca., USA). |
| 15 | Cross-Stück | Valco (Supelco SA, ausgebohrt auf ca. 0,5 mm Durchmesser). |
| 16 | Manometer | 0-600 bar (IKA, D-Staufen) für Rezykliersystem I. |
| 17 | Rezyklierpumpe | Gilson 303 (Synmedic, Heizmanschette, Ciba-Geigy). |
| 18 | UV-Detektor | Linear UVIS 200 mit SFC-Zelle und Heizschlauch, 1,4 µl Zellvolumen, 2 mm Weglänge (Henngeler, CH-Riehen). |
| 19 | Extraktionszelle | Stahlrohr mit Schraubanschlüssen. |
| 20 | Statischer Mischer | 1-3 Mischelemente (SMXE DN 3,2) (Sulzer Chemtech, CH-Winterthur) Stahlrohr mit Schraubanschluss (Ciba-Geigy). |
| 21 | Druckaufnehmer | Piezoresistiv Serie 15 Typ PA-15 (Keller AG, CH-Winterthur) Anschluss mit minimalem Totvolumen (Ciba-Geigy). |
| 22 | Druckregulator | Piezo-Druckregulator mit Piezokristall und Heizelement (EP-A-0 427 671). |
| 23 | Piezo Driver | P-864 und PZT-control E-808. (Polyscience AG, CH-Cham). |
| 24a/24b | T-Stück | SSI 01-0165 (Supelco) 24b bevorzugt ausgebohrt auf 0,5 mm Durchm. |
| 25 | Statischer Mischer | 1 Stange à 5 Stück (SMV-2 DN 10) (Sulzer Chemtech, CH-Winterthur) Stahlrohr mit Schraubanschluss (Ciba-Geigy). |
| 26 | Sammelgefäss | Dreihals-Rundkolben (50 ml). |
| 27 | Rezyklierpumpe | Schlauchquetschpumpe. (Kontron Inst.AG, CH-Zürich) Kontron Analytic LC-Pump |
| 28 | Wasserbad | mit Heizaggregat. |
| 29 | T-Stück | wie 24 |
| 30a/30b | Rückschlagsventil | wie 13, 30b bevorzugt |
| 31a/31b | Dosierpumpe | für wasserlösliche Substanz wie 7, 31b bevorzugt |
| 32a/32b | Gefäss | Vorrat mit wasserlöslicher Substanz, 32b bevorzugt |
| 33 | Statischer Mischer | wie 20 |
| A,C,D,F,G,I,J,L,N | | Dreiwege-Hähne SSI 02-0124 (Supelco). |
| B,E,H,K,M,O | | Zweiwege-Hähne SSI 02-0120 (Supelco). |

Die Pumpen 2 und 7 fördern das CO₂ und den Modifikator aus den Vorratsgefässen 1 und 6 in die Apparatur. Die CO₂-Pumpe wird vorzugsweise auf -10°C gekühlt, wobei das CO₂ eine Dichte von ca. 1 g/ml hat und sich gut pumpen lässt. Die Pumpendrucksicherung 3 zeigt den Druck der Pumpen 2 und 7 an. Der Pulsdämpfer 4 dämpft die Druckimpulse der Pumpen 2 und 7 , welche beim Zurückziehen der Pumpenkolben entstehen. Die Pumpenkontrolle 8 steuert Fluss und Phasenmischverhältnis der beiden Pumpen 2 und 7. Die statischen Mischer 9, 20, 25, 33 haben keine beweglichen Teile. Die Mischung entsteht durch Strömungen in einem Stahlrohr mit mehreren eingebauten Mischelementen (Strömungsbrechern), welche die Strömungslinien aufteilt und sammelt. Der dynamische Mischer 10 hat einen beweglichen Teil. Durch die Bewegung entsteht eine turbulente Strömung, welche die eingesetzten Phasen vermischt. Der Filter 11 hält unerwünschte Fremdpartikel in der mobilen Trägerphase bestehend aus CO₂ und Modifikator zurück. Der Injektor 12 ist für zusätzliche Modifikatorzugaben vorgesehen. Das Rückschlagsventil 13 lässt die mobile Trägerphase nur in Richtung A → B durch. Sollte der Druck in Richtung B abfallen und kleiner als in Richtung A werden, wird komprimierte Phase nachgefüllt, bis stabile, gleiche Druckbedingungen herrschen. Das einstellbare Drucksicherheitsventil 14 öffnet bei unerwünschten Überdrücken. Das Cross-Stück 15 ist in alle Richtungen geöffnet. Das Manometer 16 zeigt den Druck im Rezyklierkreislauf I an. Dieser ist durch die Anordnung C-D-19-20-F-G-18-17-15-C oder bevorzugt C-15-17-18-G-F-20-D-C definiert. Im Rezyklierkreislauf I befindet sich die komprimierte, homogene Mischphase unter homogenen Bedingungen. Die Rezyklierpumpe 17 befördert die mobile Trägerphase aus CO₂ und Modifikator durch die Extraktionszelle 19 und den statischen Mischer 20 im Rezyklierkreislauf I. Dabei werden die in der Extraktionszelle 19 vorgelegten lipophilen Bestandteile (Phospholipid und gegebenenfalls Cholesterin) gelöst. Der UV-Detektor 18 zeigt den Homogenisierungsgrad der komprimierten lipidhaltigen Mischphase mit den in der Extraktionszelle 19 vorgelegten lipophilen Bestandteilen an. Das Detektorsignal wird auf einem Schreiber aufgezeichnet. Die Extraktionszelle 19 stellt ein druckstabiles Stahlrohr mit Schraubanschlüssen und Filtern am Eingang und Ausgang dar. Dafür lassen sich auch geleerte chromatographische Säulen verwenden. Der Druckaufnehmer 21 misst den Druck hinter dem Rezyklierkreislauf I. Der Druckregulator 22 enthält einen Piezokristall, welcher vom Piezo Driver 23 gesteuert wird und dabei die erforderlichen Druckbedingungen einstellt. Der Piezo Driver 23 stellt automatisch den gewünschten Arbeitsdruck unabhängig von den Durchflussbedingungen ein. In der Anordnung 24a,b-30a,b-31a,b-32a,b lässt sich die Menge wasserlöslicher oder hydrophiler Substanz zudosieren, die in Liposomen verkapselt werden soll. Die Anordnung im Entspannungsbereich 24b-30b-31b-32b ist bevorzugt. Man kann aber auch die Anordnung im Hochdruckbereich 24a-30a-31a-32a wählen. Der statische Mischer 25 dient als Homogenisator bei der Liposomenbildung. Die Rezyklierpumpe 27 befördert die wässrige Phase aus dem Sammelgefäss 26 durch den statischen Mischer 33 in den Rezyklierkreislauf II. Dieser ist durch die Anordnung 29-34-26-27-29 definiert. Darin wird eine unkontrollierte Schaumbildung bei der Entspannung der Mischphase verhindert und Homogenität der entspannten Mischphase erzeugt. Das Wasserbad 28 sorgt für konstante Temperaturbedingungen im Rezyklierkreislauf I, im Pumpenkopf der Rezyklierpumpe 17 und in der Detektionszelle 18. Die Dreiwege-Hähne A,C,D,F,G,I,J,L,N leiten die Ströme der komprimierten Phase durch (ein Ausgang stets geöffnet) oder verteilen sie in zwei Richtungen (zwei Ausgänge geöffnet). Die Zweiwege-Hähne B,E,H,K,M,O leiten die Ströme durch oder sperren sie.

Das in der geschlossenen Apparatur gem. Fig.1 durchführbare Verfahren und die Produktströme lassen sich bei Verwendung eines repräsentativen Phospholipids und eines repräsentativen lipophilen Hilfsstoffs (Cholesterin) unter Zusatz einer wasserlöslichen Substanz anhand von folgenden Teilschritten beschreiben:
α) Die weiter vorn und in Fig.1 beschriebene geschlossene Apparatur wird mit einer Mischphase aus CO₂/Ethanol (=mobile Trägerphase) beschickt (Druckregulator: 250 bar, Temperatur Druckregulator: 90°C, Temperatur Wasserbad: 54°C, Einstellung an den Positionen 21 und 28). Die Hähne D,F,J,L und N sind geschlossen. Die Hähne A,B,C,E,G,H,I,K,M und O sind geöffnet. Die mobile Phase fliesst durch die Apparatur in Richtung A,B,C,D,E,F,G,H,I,21,G,18,17,15,C, oder alternativ A,B,C,D,15,17,18,G,H,I,21, und den Bypass A,I, aber nicht durch die Extraktionszelle 19. Der Druck wird mit dem Druckregulator 22 auf 250 bar eingestellt. Die Durchflussgeschwindigkeit der Dosierpumpe 31b ist von der Konzentration der Lipide bei der Druckentspannung der Mischphase abhängig.
β) Das Phospholipid, z.B.POPC, und Cholesterin werden in einer Extraktionszelle eingewogen (molares Verhältnis 7:3). Man baut die Extraktionszelle in Position 19 ein und hält alle Positionen nach A und vor 21 auf einer konstanten Temperatur von 60°C. Die Hähne sind in denselben Richtungen wie im Verfahrensschritt α) eingestellt.
γ) Messung der Homogenität der mobilen Trägerphase am Detektor 18. Die UV-Absorptionskurve wird mit einem Schreiber aufgenommen.
δ) Die Hähne D und F werden geöffnet und die Hahne E und H geschlossen. Alle übrigen Hähne bleiben in denselben Stellungen wie im Verfahrensschritt α). Dabei wird der Rezyklierkreislauf I: C,D,19,20,F,G,18,17,15,C oder alternativ C,15,17,18,G,F,-20,19,D,C geschlossen. Das Rückschlagsventil 13 (fakultativ) hält einen kontinuierlichen Druck im Rezyklierkreislauf I aufrecht Druckausgleich mit den Pumpen 2 und 7 über Bypass A-I und Druckregulator 22.
ε) Das in der Extraktionszelle 19 eingewogene Phospholipid mit Cholesterin löst sich bis zur Einstellung eines Gleichgewichts in der komprimierten Mischphase.
ζ) Bei stabilem Gleichgewicht (UV-Absorption stabil 18) werden die Hähne A,I,D und F geschlossen und die Pumpe 31b eingeschaltet. Die Hahne E und H werden geöffnet. Die übrigen Hähne sind in denselben Richtungen wie im Verfahrensschritt α) eingestellt.
η) Die komprimierte Mischphase enthaltend Phospholipid und Cholesterin wird aus dem Rezyklierkreislauf I durch den Druckregulator 22 entspannnt und von Position 24b in Kontakt mit der wässrigen Phase gebracht. Dabei bilden sich spontan Liposomen.
ϑ) Im Rezyklierkreislauf II bestehend aus der Anordnung 29-34-26-27-29 wird die wässrige Lipsomendispersion verdünnt und homogenisiert.
τ) Wenn die UV-Absorption das ursprüngliche Niveau der mobilen Trägerphase errreicht hat (keine erkennbare Absorption für Phospholipide) und der Absorption im Verfahrenssschritt γ) entspricht, wird das Experiment abgeschlossen und die Liposomensuspension qualitativ und quantitativ mittels HPLC analysiert. Ein Teil der Suspension wird im Lichtmikroskop betrachtet.

### Beispiel 1

Placeboversuch mit dem Phospholipid [1-n-Hexadecanoyl-2-(9-cis-octadecenoyl)-3-sn-phosphatidylcholin (POPC)] und Cholesterin. Durchführung gemäss den Arbeitssehritten α)-τ) und der in Fig.1 dargestellten Apparatur, aber ohne Anwendung des Rezyklierkreislaufs II und ohne Zudosierung von wasslöslicher Substanz. Die genannten

Positionen entsprechen den Bezugszeichen in Fig.1, welche weiter vorn beschrieben sind:
- Einwaage:: 52,86 mg POPC
8,97 mg Cholesterin.
- Mobile Trägerphase:: CO₂ und Ethanol (96 % v/v).
- Detektion:: UV-Absorption bei 210 nm.
- Durchfluss CO₂:: 0,465 ml/min an Pumpe 2.
Modifikator 0,035 ml/min an Pumpe 7.
- Druck:: vor Extraktionszelle an Position 8 und 16: ca. 300 bar.
nach Extraktionszelle an Pos.22: ca. 250 bar.
Durch den Einsatz einer Rezyklierpumpe (Kolbenpumpe-Pos. 17) entstehen Druckschwankungen von ca. 10 bar (nach Zurückziehen des Pumpenkolbens).
- Temperatur:: Wasserbad: 54°C (Pos.28).
Detektionszelle: ca. 54°C (Pos.18).
Rezyklierpumpenkopf: ca. 54°C (Pos.17). Druckregulator auf 90°C erwärmt.
- Extraktionszelle:: Typ Valco (Vorsäule) mit
350 µl Volumen.

Die Analyse mit HPLC zeigt keine Abbauprodukte in der Liposomensuspension. POPC und Cholesterin werden während des Bildungsvorgangs der Liposomen nicht abgebaut. In der erhältlichen Liposomendispersion wurden folgende Mengen gefunden:
- POPC: 29.5 mg (55,8 % Ausbeute)
- Cholesterin: 6,2 mg (69,4 % Ausbeute)
Molverhältnis POPC:Cholesterin = 3:1.

### Beispiel 2

- Einwaage:: 12,20 mg Cholesterin USP XX
59,40 mg POPC
Molverhältnis POPC zu Cholesterin: 2,5:1
0,425 mg/ml Zinkphthalocyanintetrasulfonat [ZnPc(SO₃H)₄] in dest.Wasser
- Durchfluss CO₂: 1,86 ml/min. An Pumpe 2
0,14 ml/min. An Pumpe 7
- Durchfluss=geschwindigkeit:: An Dosierpumpe 31
- 0,00 - 5,59 min.: 12,0 µl/min.
- 6,00 - 10,59 min.: 4,5 µl/min
- 11,00 - 17,00 min.: 1,5 µl/min.

Das Verfahren wird analog Beispiel 1 zu den dort angegebenen Bedingungen durchgeführt Die Analyse mit HPLC zeigt keine Abbauprodukte in der Liposomendispersion. POPC, Cholesterin und Zinkphthalocyanin-tetrasulfonat werden während des Bildungsvorgangs der Liposomen nicht abgebaut. In der erhältlichen Liposomendispersion wurden vor der Gelfiltration folgende Mengen gefunden:
- POPC:: 45,70 mg (77 % der Einwaage)
- Cholesterin:: 8,14 mg (67,0 % der Einwaage)
Molverhältnis POPC:Cholesterin = 3:1.

1,0 ml Liposomendispersion (1,03 mg Cholesterin und 5,78 mg POPC) werden gelfiltriert, um nicht eingeschlossenes ZnPc(SO₃H)₄ zu entfernen. In den Fraktionen mit Liposomen werden folgende Mengen gefunden:
- POPC:: 4,84 mg (84 % der Einwaage)
- Cholesterin:: 0,90 mg (87 % der Einwaage)
- ZnPc(SO₃H)₄: 3,6 µg, davon 2,6 µg in Liposomen.
72 % Wirkstoff eingeschlossen mit Einschlussvolumen von ca. 0,7 l/mol Lipid

## Patentansprüche

1. Verfahren zur Herstellung einer Liposomendispersion enthaltend
a) einen in Liposomen zu verkapselnden Stoff oder ein zu verkapselndes Stoffgemisch mit wasserlöslichen oder hydrophilen Eigenschaften;
b) mindestens ein Phospholipid der Formel 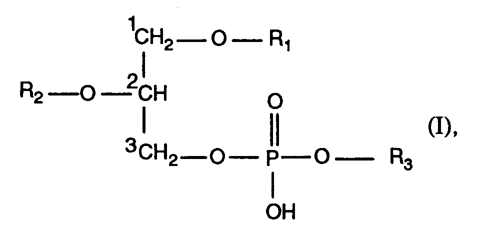 worin R₁ C₁₀₋₂₀-Acyl, R₂ Wasserstoff oder C₁₀₋₂₀-Acyl, R₃ Wasserstoff, 2-Trimethylamino-1-ethyl, 2-Amino-1-ethyl, C₁₋₄-Alkyl, C₁₋₅-Alkyl substituiert durch Carboxy, C₂₋₅-Alkyl substituiert durch Hydroxy, C₂₋₅-Alkyl substituiert durch Carboxy und Hydroxy oder C₂₋₅-Alkyl substituiert durch Carboxy und Amino bedeuten oder Salze dieser Verbindungen;
c) Wasser in der für die vorgesehene Anwendung erforderlichen Reinheit und gegebenenfalls
d) zusätzliche für die vorgesehene Anwendung übliche Hilfsstoffe, dadurch gekennzeichnet, dass man ein Gemisch bestehend aus mindestens einem Phospholipid b) und gegebenenfalls für die vorgesehene Anwendung üblichen lipophilen Hilfsstoffen d) einer mobilen Trägerphase bestehend aus Kohlendioxid und einem polaren organischen Lösungsmittel (Modifikator) unter Druck- und Temperaturbedingungen höher als dem kritischen Druck und der kritischen Temperatur einer reinen Kohlendioxidphase aussetzt, die erhältliche komprimierte Mischphase auf Normaldruck entspannt und in eine wässrige Phase enthaltend einen in Liposomen zu verkapselnden Stoff oder ein zu verkapselndes Stoffgemisch mit wasserlöslichen oder hydrophilen Eigenschaften a) und gegebenenfalls für die vorgesehene Anwendung übliche wasserlösliche Hilfsstoffe d) überführt und gegebenenfalls das organische Lösungsmittel entfernt und/oder eine Fraktion von Liposomen mit einem gewünschten Bereich des Durchmessers abtrennt und/oder die Liposomendispersion in eine für die vorgesehene Anwendung geeignete Form bringt.

2. Verfahren gemäss Anspruch 1, dadurch gekennzeichnet, dass man als zu verkapselnden Stoff oder Stoffgemisch a) radioaktive Markerverbindungen, fluoreszierende Verbindungen, pharmazeutische Wirkstoffe oder Wirkstoffkombinationen verwendet.

3. Verfahren gemäss Anspruch 2, dadurch gekennzeichnet, dass man als zu verkapselnden Stoff a) hydrophile oder wasserlösliche pharmazeutische Wirkstoffe verwendet.

4. Verfahren gemäss Anspruch 2, dadurch gekennzeichnet, dass man als zu verkapselnden Stoff a) Zinkphthalocyanin-tetrasulfonat [ZnPc(SO₃H)₄] verwendet.

5. Verfahren gemäss Anspruch 1, dadurch gekennzeichnet, dass man als Phospholipid b) synthetisches, im wesentlichen reines 1-n-Hexadecanoyl-2-(9-cis-octadecenoyl)-3-sn-phosphatidylcholin verwendet.

6. Verfahren gemäss Anspruch 1, dadurch gekennzeichnet, dass man als Komponente c) sterilisiertes Wasser für Injektionszwecke verwendet.

7. Verfahren gemäss Anspruch 1, dadurch gekennzeichnet, dass man als lipophilen Hilfsstoff d) Cholesterin verwendet.

8. Verfahren gemäss Anspruch 1, dadurch gekennzeichnet, dass man als Hilfsstoff d) wasserlösliche Hilfsstoffe zur Herstellung von isotonischen Bedingungen verwendet.

9. Verfahren gemäss einem der Ansprüche 1-8, dadurch gekennzeichnet, dass die mobile Trägerphase Kohlendioxid und Ethanol enthält.

10. Verfahren gemäss einem der Ansprüche 1-9, dadurch gekennzeichnet, dass man einen Druckbereich von ca. 72-400 bar anwendet.

11. Verfahren gemäss Anspruch 10, dadurch gekennzeichnet, dass man einen Druckbereich von ca. 200-300 bar anwendet.

12. Verfahren gemäss einem der Ansprüche 1-11, dadurch gekennzeichnet, dass man eine erhöhte Temperatur von ca. 32-100°C anwendet.

13. Verfahren gemäss Anspruch 12, dadurch gekennzeichnet, dass man eine erhöhte Temperatur von ca. 50-60°C anwendet.

14. Verfahren gemäss einem der Ansprüche 1-13, dadurch gekennzeichnet, dass man die erhältliche Lipsomendispersion in eine für die intravenöse Applikation geeignete Form bringt.

## Claims

1. A process for the preparation of a liposome dispersion containing
a) a substance for encapsulation in liposomes or a mixture of substances for encapsulation having water-soluble or hydrophilic properties;
b) at least one phospholipid of formula 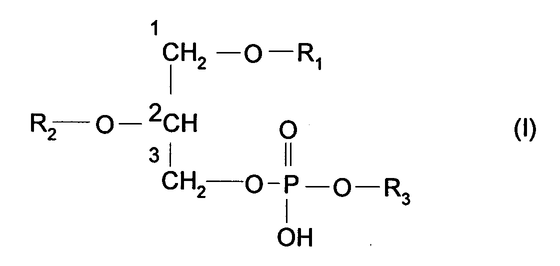 wherein
R₁ is C₁₀₋₂₀-acyl,
R₂ is hydrogen or C₁₀₋₂₀-acyl,
R₃ is hydrogen, 2-trimethylamino-1-ethyl, 2-amino-1-ethyl, C₁₋₄-alkyl, C₁₋₅-alkyl substituted by carboxy, C₂₋₅-alkyl substituted by hydroxy, C₂₋₅-alkyl substituted by carboxy and by hydroxy, or C₂₋₅₋alkyl substituted by carboxy and by amino,
or salts of these compounds;
c) water in the purity required for the intended application, and where appropriate
d) additional excipients customary for the intended application,
characterised in that a mixture consisting of at least one phospholipid b) and, where appropriate, lipophilic excipients d) customary for the intended application, is subjected to a mobile carrier phase consisting of carbon dioxide and a polar organic solvent (modifier) under pressure and temperature conditions which are higher than the critical pressure and the critical temperature of a pure carbon dioxide phase, the compressed mixed phase that is obtainable is reduced to normal pressure and is transferred to an aqueous phase containing a substance for encapsulation in liposomes or a mixture of substances for encapsulation a) having water-soluble or hydrophilic properties and, where appropriate, water-soluble excipients d) customary for the intended application, and, where appropriate, the organic solvent is removed and/or a fraction of liposomes having a desired diameter range is separated off and/or the liposome dispersion is converted into a form suitable for the intended application.

2. Process according to claim 1, characterised in that radioactive labeling compounds, fluorescent compounds, pharmaceutical active ingredients or active ingredient combinations are used as the substance or mixture of substances for encapsulation a).

3. Process according to claim 2, characterised in that hydrophilic or water-soluble pharmaceutical active ingredients are used as the substance for encapsulation a).

4. Process according to claim 2, characterised in that zinc-phthalocyanine tetrasulphonate [ZnPc(SO₃H)₄] is used as the substance for encapsulation a).

5. Process according to claim 1, characterised in that synthetic, substantially pure 1-n-hexa-decanoyl-2-(9-cis-octadecenoyl)-3-sn-phosphatidyl choline is used as the phospholipid b).

6. Process according to claim 1, characterised in that sterilised water for injection purposes is used as component c).

7. Process according to claim 1, characterised in that cholesterol is used as the lipophilic excipient d).

8. Process according to claim 1, characterised in that water-soluble excipients for the establishment of isotonic conditions are used as the excipient d).

9. Process according to one of claims 1 to 8, characterised in that the mobile carrier phase contains carbon dioxide and ethanol.

10. Process according to one of claims 1 to 9, characterised in that a pressure range of approximately 72 to 400 bar is used.

11. Process according to claim 10, characterised in that a pressure range of approximately 200 to 300 bar is used.

12. Process according to one of claims 1 to 11, characterised in that an elevated temperature of approximately 32 to 100°C is used.

13. Process according to claim 12, characterised in that an elevated temperature of approximately 50 to 60°C is used.

14. Process according to one of claims 1 to 13, characterised in that the liposome dispersion that is obtainable is converted into a form suitable for intravenous application.

## Revendications

1. Un procédé de préparation d'une dispersion de liposomes contenant
a) une substance à encapsuler dans les liposomes ou un mélange de substances à encapsuler au caractère soluble dans l'eau ou hydrophile;
b) au moins un phospholipide de formule 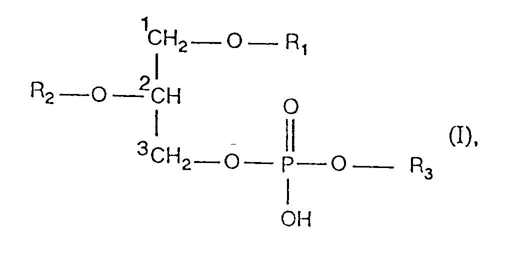 où R₁ signifie un groupe C₁₀₋₂₀-acyle, R₂ signifie l'hydrogène ou un groupe C₁₀₋₂₀-acyle, R₃ signifie l'hydrogène ou un groupe 2-triméthylamino-1-éthyle, 2-amino-1-éthyle, C₁₋₄-alkyle, C₁₋₅-alkyle substitué par un groupe carboxy, C₂₋₅-alkyle substitué par un groupe hydroxy, C₂₋₅-alkyle substitué par un groupe carboxy et hydroxy ou C₂₋₅-alkyle substitué par un groupe carboxy et amino ou les sels de ces composés;
c) de l'eau d'une pureté nécessaire pour l'utilisation envisagée et éventuellement
d) des excipients supplémentaires habituels pour l'utilisation envisagée;
procédé caractérisé en ce qu'un mélange comprenant au moins un phospholipide b) et éventuellement des excipients lipophiles d) habituels pour l'utilisation envisagée, est soumis à une phase support mobile comprenant de l'anhydride carbonique et un solvant organique polaire (modificateur) sous des conditions de pression et de température supérieures à la pression critique et à la température critique d'une phase pure d'anhydride carbonique, on réduit la phase mixte comprimée obtenue à la pression normale et on la transfère dans une phase aqueuse comprenant une substance à encapsuler dans les liposomes ou un mélange de substances à encapsuler ayant le caractère soluble dans l'eau ou hydrophile a) et éventuellement des excipients solubles dans l'eau d) habituels-pour l'application envisagée et on élimine éventuellement le solvant organique et/ou on sépare une fraction des liposomes ayant un domaine de diamère souhaité et/ou on met la dispersion de liposomes sous une forme appropriée pour l'utilisation prévue.

2. Un procédé selon la revendication 1, caractérisé en ce que comme substance ou mélange de substances à encapsuler a), on utilise des composés marqués radioactifs, des composés fluorescents, des substances actives pharmaceutiques ou des combinaisons de substances actives.

3. Un procédé selon la revendication 2, caractérisé en ce que comme substance à encapsuler a), on utilise des substances actives pharmaceutiques hydrophiles ou solubles dans l'eau.

4. Un procédé selon la revendication 2, caractérisé en ce que comme substance à encapsuler a), on utilise le tétrasulfonate de phtalocyanine de zinc [ZnPc(SO₃H)₄].

5. Un procédé selon la revendication 1, caractérisé en ce que comme phospholipide b), on utilise la 1-n-hexadécanoyl-2-(9-cis-octadécénoyl)-3-sn-phosphatidylcholine synthétique substantiellement pure.

6. Un procédé selon la revendication 1, caractérisé en ce que comme composant c), on utilise de l'eau stérilisée pour injection.

7. Un procédé selon la revendication 1, caractérisé en ce que comme excipient lipophile d), on utilise du cholestérol.

8. Un procédé selon la revendication 1, caractérisé en ce que comme excipient d), on utilise des excipients solubles dans l'eau pour l'établissement de conditions isotoniques.

9. Un procédé selon l'une quelconque des revendications 1 à 8, caractérisé en ce que la phase support mobile contient de l'anhydride carbonique et de l'éthanol.

10. Un procédé selon l'une quelconque des revendications 1 à 9, caractérisé en ce qu'on utilise un intervalle de pression d'environ 72 à 400 bar.

11. Un procédé selon la revendication 10, caractérisé en ce qu'on utilise un intervalle de pression d'environ 200 à 300 bar.

12. Un procédé selon l'une quelconque des revendications 1 à 11, caractérisé en ce qu'on utilise une température élevée d'environ 32 à 100°C.

13. Un procédé selon la revendication 12, caractérisé en ce qu'on utilise une température élevée d'environ 50 à 60°C.

14. Un procédé selon l'une quelconque des revendications 1 à 13, caractérisé en ce qu'on met la dispersion de liposomes obtenue sous une forme appropriée pour l'administration par voie intraveineuse.
